# EUROPEAN PATENT APPLICATION

(11) **EP 1 974 674 A1**
(43) Date of publication of application: **01.10.2008**
(21) Application number: 06832888.9
(22) Date of filing: 17.11.2006
(51) Int. Cl.: A61B 10/00, A61B 1/04, A61B 5/107, A61B 19/00

(54) **IMAGING SYSTEM**

(30) Priority: 20.01.2006 JP 2006013028
(71) Applicant: Sumitomo Electric Industries, Ltd., Osaka-shi, Osaka 541-0041 (JP)
(72) Inventor: HASEGAWA, Takemi, Yokohama-shi, Kanagawa 2448588 (JP); IWASAKI, Takashi, Itami-shi, Hyogo 6640016 (JP); OKUNO, Toshiaki, Yokohama-shi, Kanagawa 2448588 (JP); ONISHI, Masashi, Yokohama-shi, Kanagawa 2448588 (JP)
(74) Representative: HOFFMANN EITLE
(86) International application number: PCT/JP2006/323010
(87) International publication number: WO 2007/083437

(57) **Abstract**

An imaging system 1 comprises: an illumination light source unit 10 that emits an illumination light having a wavelength in a near-infrared range; an illumination optical system 20 that illuminates an observed object 90 with the illumination light emitted from the illumination light source unit 10; an imaging optical system 30 that guides as a physical body light the illumination light that has been illuminated by the illumination optical system 20 onto the observed object 90 and has been scattered, reflected, or refracted thereby; and an imaging unit 40 that has an imaging sensitivity in a wavelength band of a near-infrared range, receives, the physical body light arrived after being guided by the imaging optical system 30, and images the image of the observed object 90. The imaging unit 40 receives the physical body light after the light has passed through water and hemoglobin.

## Description

### Technical Field

The present invention relates to an imaging system that can be suitably used for observing and recording a surgical field or the inside of a blood vessel.

### Background Art

Imaging systems disclosed in Patent Documents 1, 2 are known as being suitable for observing and recording a surgical field or the inside of a blood vessel. The imaging system disclosed in Patent Document 1 comprises a mechanism for switching a flow rate of a transparent fluid in a blood vessel endoscope and is designed for performing safe optical observations inside the blood vessel by removing blood from a view field with a high-speed fluid and then preventing blood from penetrating into the view field with a low-speed fluid. In the imaging system disclosed in Patent Document 2, a lens is held in a holding tool for converging an illumination light for illuminating the surgical field onto the surgical field and the imaging system is design to take bright images by imaging the surgical field with an imaging unit via the lens.
[Patent Document 1] US Patent No. 5,053,002 specification
[Patent Document 2] US Patent No. 5,803,905 specification

### Disclosure of the Invention

### [Problems Addressed by the Invention]

However, with the imaging system disclosed in Patent Document 1, the invasion degree of the observed object is high and long-term observations are difficult. Furthermore, a transparent fluid is injected into the blood vessel to prevent blood from shielding the view field. As a result the internal pressure of the blood vessel is increased and blood composition is changed. On the other hand, with the imaging system disclosed in Patent Document 2, the view field is restricted in the surgical fields with bleeding,. Furthermore, in the surgical fields with bleeding, the view field is obstructed by blood. Thus, none of the imaging systems disclosed in Patent Documents 1, 2 is suitable for medical applications. The present invention has been created to resolve these problems, and it is an object thereof to provide an imaging system that can be suitably used for medical applications.

### [Means to Resolve the Problems]

The imaging system in accordance with the present invention is an imaging system suitable for imaging an observed object located in blood, this imaging system comprising an illumination light source unit that emits an illumination light having a wavelength in a near-infrared range, an illumination optical system that illuminates the observed object with the illumination light emitted from the illumination light source unit, an imaging optical system that guides as a physical body light the illumination light that has been illuminated by the illumination optical system onto the observed object and has been scattered, reflected, or refracted thereby, and an imaging unit that has an imaging sensitivity in a wavelength band of a near-infrared range, receives the physical body light arrived after being guided by the imaging optical system, and images the observed object.

In the imaging system in accordance with the present invention, an illumination light having a wavelength in a near-infrared range is emitted from the illumination light source unit. Where the observed object is illuminated with the illumination light emitted by the illumination light source unit by means of the illumination optically system, the illumination light that is scattered, reflected, and refracted in the observed object is generated as a physical body light. The physical body light generated in the observed object is guided by the imaging optical system to the imaging unit that has an imaging sensitivity in the wavelength band in a near-IR region, and an image of the observed object (region of interest thereof) is imaged with the imaging unit. At this time, the physical body light that passed through water and hemoglobin is received by the imaging unit. Even when the region of interest of the observed object is covered with blood, because the illumination light and physical body light pass through blood, the imaging system can be advantageously used for medical applications.

In the imaging system in accordance with the present invention, the illumination light emitted by the illumination light source unit preferably includes a light with a wavelength within a range of 0.9 µm to 1.3 µm or 1.5 µm to 1.8 µm, more preferably includes a light with a wavelength within a range of 1.2 µm to 1.3 µm or 1.6 µm to 1.8 µm. In this case, it is especially preferred that the illumination light and physical body light be transmitted by blood with low loss.

Further, it is preferred that the illumination light emitted by the illumination light source unit include a light with a wavelength within a range of 0.9 µm to 1.1 µm, and the imaging unit include a CCD comprising silicon. A CCD comprising silicon is inexpensive and has an imaging sensitivity in a wavelength band with a wavelength of 1.1 µm and less. Therefore, the physical body light can be imaged by using an inexpensive CCD. Further, by using the long-wavelength light with a wavelength of 0.9 µm to 1.1 µm from the wavelength band with a wavelength of 1.1 µm and less, the scattering can be decreased and observation sensitivity can be increased.

In the imaging system in accordance with the present invention, the illumination light source unit preferably comprises: a pulse laser beam source that outputs a pulse light; and an optical fiber that couples the pulse light outputted from the pulse laser beam source with a HE 11 mode and outputs as the illumination light the pulse light whose spectrum is expanded by a nonlinear optical effect. In this case, the illumination light emitted from the illumination light source unit is preferably a broadband super continuum light. When the observed object has an absorption light matching the wavelength of the illumination light, there is a risk of the observed object generating heat. Therefore, it is preferred that an illumination light with a diffused spectrum such as SC light be used.

In the imaging system in accordance with the present invention, it is preferred that at least one of the illumination light source unit and the imaging unit include an element (for example, a light-emitting element or a two-dimensional imaging element) comprising an InGaAs material, or an element comprising an Extended-InGaAs material, or an element comprising a GaInNAsSb material. In this case, a material with a sensitivity in near-infrared range is preferred. It is preferred that the imaging unit be a two-dimensional imaging element. Where an element comprising an InGaAs material is used, light with a wavelength of less than 1.7 µm can be emitted or received. Where an element comprising an Extended-InGaAs material is used, light with a wavelength of 1.7 µm or more can be emitted or received. Where an element comprising an Extended-InGaAs material is used, light with a wavelength of 1.7 µm or more to 2.65 µm or less can be emitted or received at room temperature. Where an element comprising a GaInNAsSb material is used, noise can be suppressed to a low level and a bright image can be acquired in a wavelength range of 1.7 to 3.0 µm. One of the reasons therefor is that the GaInNAsSb material is lattice matched to InP.

In the imaging system in accordance with the present invention, the illumination optical system preferably comprises a shadowless lamp reflection mirror that reflects the illumination light emitted from the illumination light source unit and illuminates the observed object. Such a configuration is preferred, for example, for imaging an image of an observed object in a surgical field.

In the imaging system in accordance with the present invention, it is preferred that the illumination optical system comprise an optical fiber for illumination for guiding the illumination light emitted from the illumination light source unit to the observed object, that the imaging optical system comprise an optical fiber for imaging for guiding the physical body light generated by the observed object to the imaging unit, and the optical fiber for illumination and the optical fiber for imaging be provided inside an endoscope. Such a configuration is preferred, for example, for imaging images of the inner walls of a blood vessel.

The imaging system in accordance with the present invention preferably further comprises an optical filter, disposed between the illumination light source unit and the observed object, for transmitting a light of a predetermined wavelength of the illumination light emitted from the illumination light source unit. In this case, the observed object can be selectively illuminated with the light of a desired wavelength. Further, the imaging system in accordance with the present invention preferably further comprises an optical filter, disposed between the observed object and the imaging unit, for transmitting the light of a predetermined wavelength of the physical body light generated by the observed object. In this case, the imaging unit can receive selectively the light of a predetermined wavelength.

### [Effect of the Invention]

The present invention can provide an imaging system that can be advantageous used for medical applications.

### Brief Description of the Drawings

Fig. 1 is a structural drawing of the imaging system 1 of the first embodiment.
Fig. 2 is a structural drawing illustrating the illumination light source unit 10 of a modification example.
Fig. 3 is a structural drawing illustrating the illumination light source unit 10 of a modification example.
Fig. 4 is a structural drawing of the imaging system 2 of the second embodiment.

### [Explanation of Reference Numerals]

1, 2 - imaging system, 10 - illumination light source unit, 11 - pulse laser beam source, 12 - optical fiber, 20 - optical system for illumination, 21 a, 21 b - optical fibers for illumination, 22a, 22b - reflecting mirrors, 23a, 23b - shadowless lamp reflecting mirrors, 24a, 24b - lenses, 25 - reflecting mirror, 30 - imaging optical system, 31 - imaging optical fiber, 34 - lens, 40 - imaging unit, 50 - display unit, 60 - endoscope, 90 - observed object, 91 - region of interest, 92 - incision section, 93 - blood, 94 - blood vessel, 95 - region of interest, 96 - blood, 100, 100a, 100b, - optical filters.

### Best Modes for Carrying Out the Invention

The best mode for carrying out the present invention will be described below in greater detail with reference to the appended drawings. In the drawings, identical or analogous elements are assigned with identical reference symbols and redundant explanation thereof is omitted.

(First embodiment)

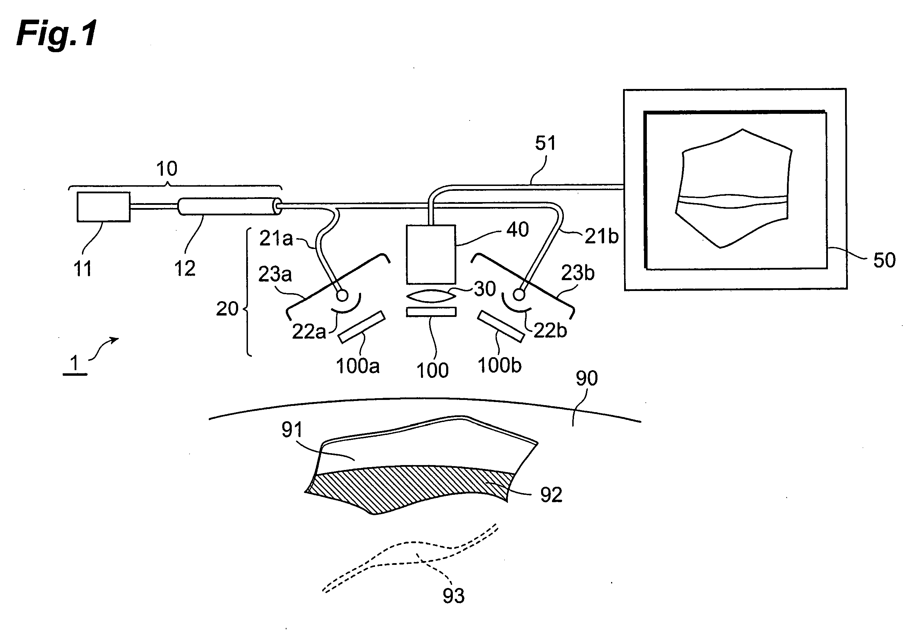

The first embodiment of an imaging system of the present invention will be descried below. Fig. 1 is a structural drawing of an imaging system 1 of the first embodiment. The imaging system 1 shown in the figure comprises an illumination light source unit 10, an illumination optical system 20, an imaging optical system 30, an imaging unit 40, and a display unit 50. The imaging system 1 is advantageously suitable for observing a region 91 of interest of an observed object 90 that can be a surgical object.

The illumination light source unit 10 emits illumination light having a wavelength in the near-IR range and includes, for example, a pulse laser beam source 11 and an optical fiber 12 with a high degree of nonlinearity. The pulse laser beam source 11 outputs pulse light with a power of 10 to 100 mW and a wavelength of 1.55 µm. The outputted pulse light is coupled to a HE11 mode of the optical fiber 12. The optical fiber 12 has a nonlinear coefficient gamma of 20 [W⁻¹km⁻¹] (preferably 30 [W⁻¹km⁻¹]) at a wavelength of the pulse light and also has a wavelength dispersion slope with an absolute value of 0.03 [ps/nm²/km] or less. As a result, the spectrum of the pulse light is expanded due to nonlinear optical effects in the optical fiber 12, and a broadband light is known as a super continuum light (SC light) is generated in the optical fiber 12. The SC light preferably has an optical power of a spectral density of 1 µW/nm or more over a wavelength range of 0.9 µm to 3.0 µm, preferably a wavelength range of 1.2 µm to 1.8 µm. The optical fiber 12 outputs the SC light as the illumination light.

A laser diode having an emission wavelength of 0.9 to 1.1 µm, or 1.3 µm, or 1.5 to 1.7 µm, a Nd:YAG laser or Yb-doped fiber laser having an emission wavelength at 1.06 µm, an Er-doped fiber laser having an emission wavelength at 1.55 µm, and a Raman laser excited by these lasers can be used to produce the illumination light. In the case where the power of the laser beam is concentrated at a specific wavelength, when the observed object 90 has an absorption line matching this wavelength, there is a risk of the observed object 90 generating heat. Therefore, it is preferred that illumination light with a diffused spectrum, such as the SC light, be used.

The illumination light source unit 10 may also include a light source, for example, a laser diode (LD), a LED, or a super-luminescence diode (SLD). In this case, the cost of the illumination light source unit 10 can be reduced and the configuration of the illumination light source unit 10 can be simplified. The illumination light source unit 10 may also be a LED that emits light, for example, having a peak wavelength in a wavelength range of 1.2 to 1.4 µm or 1.5 to 1.9 µm and a full width at half maximum of 5 nm or more. The illumination light source unit 10 may also be a laser diode, for example, having a peak wavelength within a wavelength range of 1.2 to 1.4 µm or 1.5 to 1.9 µm.

The illumination light source unit 10 preferably includes a light-emitting element comprising an InGaAs material or Extended-InGaAs or GaInNAsSb material. If a light-emitting element comprising an InGaAs material is used, light with a wavelength less than 1.7 µm can be emitted. If a light-emitting element comprising an Extended-InGaAs material is used, light with a wavelength of 1.7 µm or more to 2.65 µm or less can be obtained. The Extended-InGaAs materials are described in detail, for example, in IPRM (InP wavelength Related Material) 2003. A plurality of InAsP step layers can be grown on an InP substrate and an Extended-InGaAs layer can be grown on the InAsP buffer layer.

For example, when a laser beam source is used as the light source, the illumination light source unit 10 preferably comprises coherence decreasing means for decreasing the coherence of laser beam emitted from the laser diode. In this case, the appearance of interference or speckles of the laser beam can be suppressed and, therefore, a low-noise signal can be obtained. As a result, highly accurate image data can be obtained. For example, an integration sphere or a diffusion plate can be used as the coherence decreasing means. Furthermore, a method for realizing the decrease in coherency by using multiple illumination with a plurality of light source units can be also used. The illumination light source unit 10 may also comprise a light modulator for a high-speed modulation of the laser beam emitted form the laser diode. In this case, it is possible to use the imaging unit 40 that can detect only the laser light with a speed lower than that high-speed modulated laser beam, and the coherence can be reduced by performing time averaging of the signal detected in the imaging unit 40.

Further, it is preferred that the illumination light include light with a wavelength within a range of 0.9 µm to 1.1 µm and that the imaging unit 40 be a CCD comprising silicon. The CCD comprising silicon is inexpensive and has an imaging sensitivity in a wavelength range of 1.1 µm or less. Furthermore, even within a wavelength range of 1.1 µm and below, the scattering can be deceased and observation depth can be increased by using light with a long wavelength of 0.9 µm to 1.1 µm.

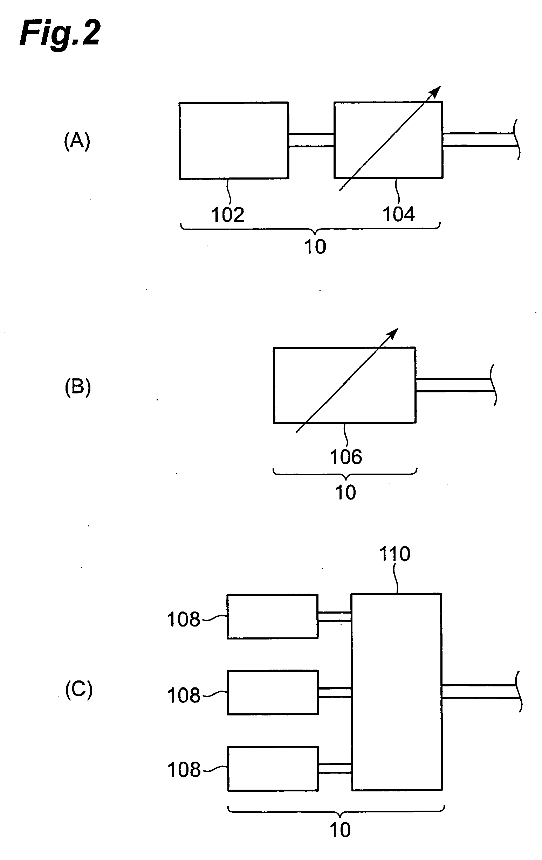

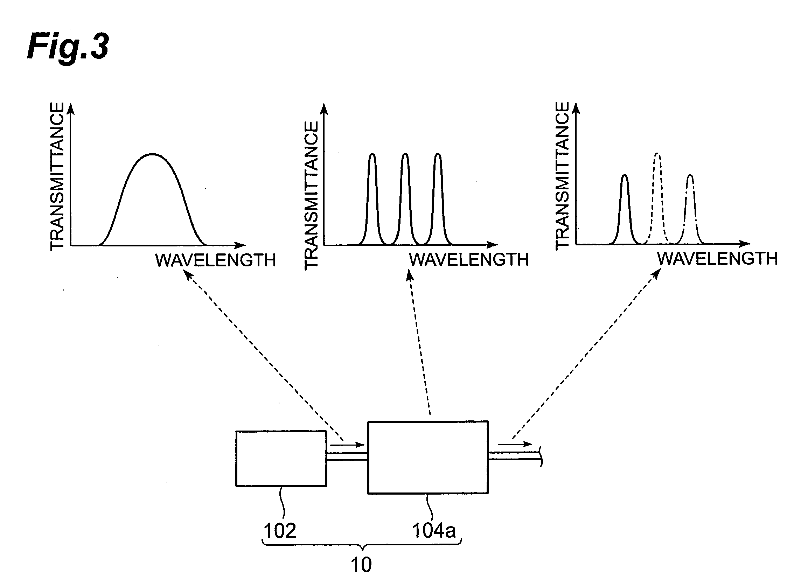

(A) to (C) of Fig. 2 and Fig. 3 are structural drawings illustrating the illumination light source unit 10 of a modification example, As shown in (A) of Fig. 2, the illumination light source unit 10 may comprise a LED 102, and a wavelength-variable filter 104 that is optically coupled to the LED 102. In this case, the wavelength band of light emitted from the LED 102 can be changed to a predetermined wavelength and (for example, 1.2 to 1.3 µm or 1.6 to 1.8 µm) at a low cost and in an easy manner. A wavelength-fixed filter that transmits light in a predetermined wavelength range (for example, a wavelength of 1.2 to 1.3 µm) may be used instead of the wavelength-variable filter 104. Furthermore, a filter 104a that simultaneously outputs three wavelengths with mutually different central wavelengths may be also used instead of the wavelength-variable filter 104 (see Fig. 3). The filter 104a may be obtained by sequentially connecting band-pass filters or combining a low-pass filter and a high-pass filter to produce the three wavelengths. When the filter 104a is a wavelength-variable filter, the values of the central wavelengths may be shifted, while maintaining the fixed distance between the central wavelengths of the three outputted wavelengths, or each central wavelength may be moved independently. By allocating the three divided wavelengths to three primary colors (RGB) of vision, the three wavelengths can be directly viewed separately due to the difference in color when an image of a visible region is displayed.

As shown in (B) of Fig. 2, the illumination light source unit 10 may include, for example, a wavelength-variable laser diode 106. For example, a laser diode of an external resonance type can be used as the wavelength-variable diode 106. In this case, light with a plurality of wavelengths having a high power is emitted from the wavelength-variable laser diode 106 and, therefore, a signal with a high SN ratio for each of a plurality of wavelengths can be obtained. The wavelength of the light emitted from the wavelength-variable laser diode 106 is in a predetermined wavelength range (for example, 1.2 to 1.3 µm or 1.6 to 1.8 µm). Furthermore, the wavelength-variable laser diode 106 may include a laser diode and a temperature regulator for regulating the temperature of the laser diode. If the temperature of the laser diode changes, the wavelength of the light emitted from the laser diode usually shifts by several nanometers.

As shown in (C) of Fig. 2, the illumination light source unit 10 may include a plurality of laser diodes 108 emitting laser beams of a plurality of wavelengths and an optical synthesizer 110 that synthesizes a plurality of laser beams emitted from a plurality of laser diodes 108. In this case, because the illumination light emitted from the illumination light source unit 10 has a plurality of wavelengths, a plurality of image data each corresponding to respective wavelength can be obtained. By using the plurality of image data, the imaging accuracy of the observed object 90 can be increased. Examples of suitable optical synthesizers 110 include a WDM coupler and an optical multiplexer. Laser beams with mutually different wavelengths (for example, (1) a combination of wavelength 1.2 µm, wavelength 1.3 µm, and wavelength 1.6 µm, and (2) a combination of wavelength 1.3 µm, wavelength 1.6 µm, and wavelength 1.8 µm) are emitted from respective laser diodes 108. The illumination light source unit 10 may also contain no optical synthesizer 110. In this case, a plurality of laser beams emitted from a plurality of laser diodes 108 are illuminated mutually independently on the observed object 90 via a plurality of optical fibers. In particular, three laser diodes 108 outputting laser beams of mutually different wavelengths (narrow-band LED or SLD may be used instead of the laser diodes 108) are preferably arranged side by side. In this case, similarly to the illumination light source unit 10 shown in Fig. 3, by allocating the three divided wavelengths to three primary colors (RGB) of vision, the three wavelengths can be directly viewed separately due to the difference in color when an image of a visible region is displayed.

Again referring to Fig. 1, the illumination optical system 20 projects the illumination light emitted from the illumination light source unit 10 onto the observed object 90 and comprises optical fibers 21a, 21b for illumination, reflecting mirrors 22a, 22b, and shadowless lamp reflecting mirrors 23a, 23b. The optical fibers 21a, 21b for illumination may be optical fibers with a high degree of nonlinearity, similar to the optical fiber 12 with a high degree of nonlinearity. In this case, the optical fiber 12 with a high degree of nonlinearity becomes unnecessary. The illumination light emitted from the illumination light source unit 10 is inputted into the incoming ends of optical fibers 21a, 21b for illumination, guided therein, and outputted from the outgoing ends thereof. The illumination light outputted from the outgoing end of one optical fiber 21 a for illumination is reflected by the reflecting mirror 22a, then reflected by the shadowless lamp reflecting mirror 23a, and irradiated on a surgical field of the observed object 90. The illumination light outputted from the outgoing end of the other optical fiber 21b for illumination is reflected by the reflecting mirror 22b, then reflected by the shadowless lamp reflecting mirror 23b, and irradiated on the surgical field of the observed object 90. Well-known surgical shadowless lamps can be used for the shadowless lamp reflecting mirrors 23a, 23b. Further, it is preferred that a conventional visible illumination light source that outputs another visible light for illumination be further provided and the reflecting mirrors be commonly used for the visible illumination with the conventional shadowless lamp and the illumination light of the present embodiment. In such a case, the user can perform blood transmission observations by natural senses similarly to the usual naked eye observations.

In the observed object 90 located in the surgical field, a region 91 of interest (ROI) such as an internal organ that is being observed is present in an incision section 92, but the region 91 of interest is covered with blood 93. Water and hemoglobin are the main light absorbents present in blood, but the illumination light that is emitted from the illumination light source unit 10 and irradiated via the illumination optical system 20 has a wavelength of 0.9 µm to 1.3 µm or 1.5 µm to 1.8 µm and is effectively transmitted through these light absorbents. As a result, the illumination light can pass through the blood 93 and illuminate the region 91 of interest, and the illumination light that was reflected, scattered, or refracted in the region 91 of interest of the observed object 90 passes through via the blood 93 and again goes out as physical body light.

The imaging optical system 30 guides the illumination light that was irradiated on the observed object 90 and scattered, reflected, or refracted thereby to the imaging unit 40 as a physical body light, and an image of the region 91 of interest is formed on the imaging surface of the imaging unit 40. The imaging unit 40 has an imaging sensitivity in a near-IR wavelength band, receives the physical body light that was guided thereto by the imaging optical system 30, and images the region 91 of interest of the observed object 90. The imaging unit 40 preferably has an especially high sensitivity at a wavelength of 0.9 µm to 1.8 µm and preferably includes a two-dimensional imaging element comprising an InGaAs material, an Extended-InGaAs material, or a GaInNAsSb material. If a two-dimensional imaging element comprising an InGaAs material is used, then light with a wavelength of less than 1.7 µm can be received. If a two-dimensional imaging element comprising an Extended-InGaAs material is used, then light with a wavelength of 1.7 µm or more to 2.65 µm or less can be received.

If necessary, an optical filter 100 may be disposed between the imaging unit 40 and observed object 90. The optical filter 100 transmits light of a predetermined wavelength of the physical body light generated by the observed object 90. As a result, the imaging unit 40 can selectively receive the light of a desired wavelength. It is preferred that the transmission wavelength band of the optical filter 100 be variable. In this case, different information relating to the observed object 90 can be extracted according to each wavelength band. Further, the optical filter 100 preferably transmits light of a plurality of predetermined wavelengths. For example, a grating can be used as the optical filter 100. Further, optical filters 100a, 100b similar to the optical filter 100 may be disposed between the illumination light source unit 10 and observed object 90. In this case, because the optical filters 100a, 100b transmit the light of a predetermined wavelength of the illumination light emitted from the illumination light source unit 10, the observed object 90 can be selectively irradiated with the light of the desired wavelength. The optical filters 100, 100a, 100b are preferred in the case where the illumination light or physical body light is a SC light. However, the optical filters 100, 100a, 100b may be also used when the illumination light is emitted from a LED.

The image data of the region 91 of interest of the observed object 90 that were obtained by the imaging process are transmitted by the imaging unit 40 to the display unit 50 via a cable 51. The display unit 50 displays the image of the region 91 of interest of the observed object 90. As a result, the user can observe on the display unit 50 the region 91 of interest that is located under the blood 93 and cannot be observed with a naked eye or by visible light imaging.

(Second embodiment)

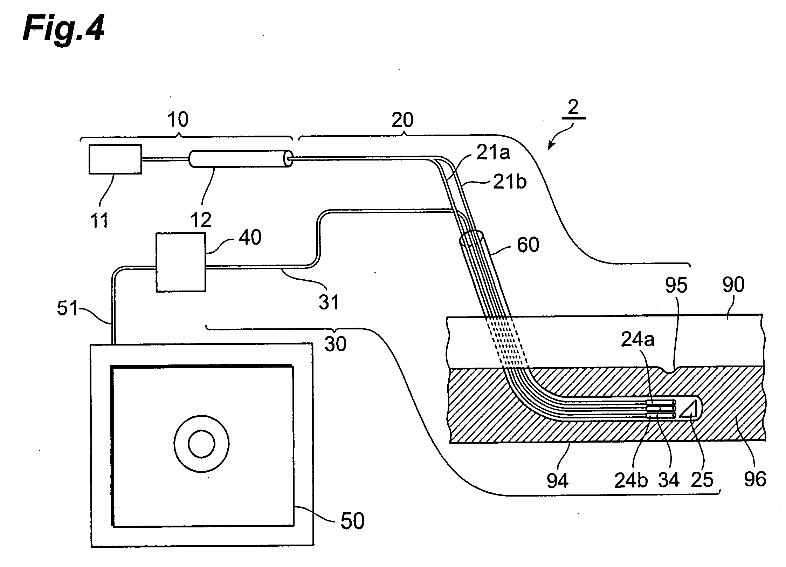

The second embodiment of the imaging system of the present invention will be described below. Fig. 4 is a structural drawing of an imaging system 2 of the second embodiment. The imaging system 2 shown in the figure comprises an illumination light source unit 10, an illumination optical system 20, an imaging optical system 30, an imaging unit 40, and a display unit 50 and can be advantageous used for observing internal walls of a blood vessel 94 of an observed object 90.

The difference between the imaging system 2 of the second embodiment and the imaging system of the first embodiment is in that the imaging optical system 30 comprises an optical fiber 31 for imaging that serves to guide the physical body light to the imaging unit 40, in that the optical fibers 21a, 21b for illumination and an optical fiber 31 for imaging are located inside an endoscope 60, and in that lenses 24a, 24b, 34 and reflective mirror 25 are provided at respective distal ends of the optical fibers 21a, 21b for illumination and optical fiber 31 for imaging.

The illumination light outputted from outgoing ends of the optical fibers 21a, 21b for illumination in the distal end section of the endoscope 60 is collected by lenses 24a, 24b, reflected at a right angle with the reflective mirror 25, and irradiated on the observed object 90. The endoscope 60 is a blood vessel endoscope that is used inside the blood vessel 94 of the observed object 90. The axes of lenses 24a, 24b, endoscope 60, and blood vessel 94 are parallel to each other in the distal end portion of the endoscope 60 inserted into the blood vessel 94. Therefore, the illumination light outgoing from the lenses 24a, 24b is reflected at almost 90° by the reflecting mirror 25 and directed toward a region 95 of interest of the inner wall of the blood vessel 94.

The inside of the blood vessel 94 is filled with blood 96, but as was described in the first embodiment, the illumination light passes through the blood 96 and illuminates the region 95 of interest, and the physical body light generated by the region 95 of interest is transmitted via the blood 96. The physical body light from the region 95 of interest is transmitted via the reflecting mirror 25 and lens 34 and forms an image on the end surface of the optical fiber 31 for imaging, which is an image band optical fiber. The optical fiber 31 for imaging transmits the image of the region 95 of interest to the imaging surface of the imaging unit 40. Similarly to the first embodiment, in the second embodiment, the imaging unit 40 transmits the image data of the region 91 of interest of the observed object 90 obtained in the imaging process to the display unit 50 via an electric cable 51. The display unit 50 displays the image of the region 95 of interest of the observed object 90. Further, in the imaging system 2, an optical filter 100 may be disposed in the middle section of the optical fiber 31 for imaging.

## Claims

1. An imaging system suitable for imaging an observed object located in blood,
the imaging system comprising:
an illumination light source unit that emits an illumination light having a wavelength in a near-infrared range;
an illumination optical system that illuminates the observed object with the illumination light emitted from the illumination light source unit;
an imaging optical system that guides as a physical body light the illumination light that has been illuminated by the illumination optical system onto the observed object and has been scattered, reflected, or refracted thereby; and
an imaging unit that has an imaging sensitivity in a wavelength band of a near-infrared range, receives the physical body light arrived after being guided by the imaging optical system, and images the observed object.

2. The imaging system according to claim 1, wherein the illumination light emitted by the illumination light source unit includes a light with a wavelength within a range of 0.9 µm to 1.3 µm or 1.5 µm to 1.8 µm.

3. The imaging system according to claim 1, wherein the illumination light emitted by the illumination light source unit includes a light with a wavelength within a range of 0.9 µm to 1.1 µm, and
the imaging unit includes a CCD comprising silicon.

4. The imaging system according to claim 1, wherein
the illumination light source unit comprises:
a pulse laser beam source that outputs a pulse light; and
an optical fiber that couples the pulse light outputted from the pulse laser beam source with a HE11 mode and outputs as the illumination light the pulse light whose spectrum is expanded by a nonlinear optical effect.

5. The imaging system according to claim 1, wherein at least one of the illumination light source unit and the imaging unit includes an element comprising an InGaAs material.

6. The imaging system according to claim 1, wherein at least one of the illumination light source unit and the imaging unit includes an element comprising an Extended-InGaAs material.

7. The imaging system according to claim 1, wherein at least one of the illumination light source unit and the imaging unit includes an element comprising a GaInNAsSb material.

8. The imaging system according to any one of claims 5 to 7, wherein the imaging unit is a two-dimensional imaging element.

9. The imaging system according to claim 1, wherein the illumination optical system comprises a shadowless lamp reflection mirror that reflects the illumination light emitted from the illumination light source unit and illuminates the observed object.

10. The imaging system according to claim 1, wherein
the illumination optical system comprises an optical fiber for illumination for guiding the illumination light emitted from the illumination light source unit to the observed object;
the imaging optical system comprises an optical fiber for imaging for guiding the physical body light generated by the observed object to the imaging unit; and
the optical fiber for illumination and the optical fiber for imaging are provided inside an endoscope.

11. The imaging system according to claim 1, further comprising an optical filter, disposed between the illumination light source unit and the observed object, for transmitting a light of a predetermined wavelength of the illumination light emitted from the illumination light source unit.

12. The imaging system according to claim 1, further comprising an optical filter, disposed between the observed object and the imaging unit, for transmitting a light of a predetermined wavelength of the physical body light generated by the observed object.
